# EUROPEAN PATENT APPLICATION

(11) **EP 2 808 385 A1**
(43) Date of publication of application: **03.12.2014**
(21) Application number: 12861317.1
(22) Date of filing: 28.12.2012
(51) Int. Cl.: C12N 15/09, C12Q 1/68

(54) **METHOD FOR DETERMINING HLA-A*24 GROUP**

(30) Priority: 28.12.2011 JP 2011289336
(71) Applicant: Toppan Printing Co., Ltd., Tokyo 110-8560 (JP)
(72) Inventor: MAKINO Yoichi, Tokyo 110-8560 (JP); YAMANE Akio, Tokyo 110-8560 (JP); AZUMA Tomohiko, Tokyo 110-8560 (JP); NAKAYAMA Masato, Tokyo 110-8560 (JP)
(74) Representative: TBK
(86) International application number: PCT/JP2012/084130
(87) International publication number: WO 2013/100139

(57) **Abstract**

According to the present invention, a method for determining the typing of an HLA-A*24 group with less ambiguity in a simple manner is provided. The method of the present invention is a method for determining an HLA-A*24 group, which includes determining the HLA-A*24 group on the basis of the results of the typing of (a) a 211st base and (b) a 395th base, in which A (adenine) in the initiation codon for an HLA gene is defined as the 1st base in the genomic DNA of a test subject. In the method for determining an HLA-A*24 group, the HLA-A*24 group is determined, further on the basis of the results of the typing of (c) at least one base selected from the group consisting of a 437th base, a 441st base, a 443rd base, a 444th base, a 447th base, and a 449th base.

SEQUENCE LISTING
<110>  TOPPAN Printing Co., Ltd.
<120>  Method for typing HLA-A*24 group
<130>  PC-16501
<150>\201@JP2011-289336
<151>\201@2011-12-28
<160>  14
<170>  PatentIn version 3.1

<210>  1
<211>  3502
<212>  DNA
<213>  Homo sapiens
<400>  1
cagaagcaga ggggtcaggg cgaagtccca gggccccagg cgtggctctc agggtctcag      60
gccccgaagg cggtgtatgg attggggagt cccagccttg gggattcccc aactccgcag     120
tttcttttct ccctctccca acctatgtag ggtccttctt cctggatact cacgacgcgg     180
acccagttct cactcccatt gggtgtcggg tttccagaga agccaatcag tgtcgtcgcg     240
gtcgctgttc taaagtccgc acgcacccac cgggactcag attctcccca gacgccgagg     300
atggccgtca tggcgccccg aaccctcgtc ctgctactct cgggggccct ggccctgacc     360
cagacctggg caggtgagtg cggggtcggg agggaaacgg cctctgcggg gagaagcaag     420
gggcccgcct ggcgggggcg caagacccgg gaagccgcgc cgggaggagg gtcgggcggg     480
tctcagccac tcctcgtccc caggctccca ctccatgagg tatttctcca catccgtgtc     540
ccggcccggc cgcggggagc cccgcttcat cgccgtgggc tacgtggacg acacgcagtt     600
cgtgcggttc gacagcgacg ccgcgagcca gaggatggag ccgcgggcgc cgtggataga     660
gcaggagggg ccggagtatt gggacgagga gacagggaaa gtgaaggccc actcacagac     720
tgaccgagag aacctgcgga tcgcgctccg ctactacaac cagagcgagg ccggtgagtg     780
accccggccc ggggcgcagg tcacgacccc tcatccccca cggacggccc gggtcgccca     840
cagtctccgg gtccgagatc caccccgaag ccgcgggacc ccgagaccct tgccccggga     900
gaggcccagg cgccttaacc cggtttcatt ttcagtttag gccaaaaatc cccccgggtt     960
ggtcggggcc gggcggggct cgggggactg ggctgaccgc ggggtcgggg ccaggttctc    1020
acaccctcca gatgatgttt ggctgcgacg tggggtcgga cgggcgcttc ctccgcgggt    1080
accaccagta cgcctacgac ggcaaggatt acatcgccct gaaagaggac ctgcgctctt    1140
ggaccgcggc ggacatggcg gctcagatca ccaagcgcaa gtgggaggcg gcccatgtgg    1200
cggagcagca gagagcctac ctggagggca cgtgcgtgga cgggctccgc agatacctgg    1260
agaacgggaa ggagacgctg cagcgcacgg gtaccagggg ccacggggcg cctacctgat    1320
cgcctgtagg tctcccgggc tggcctccca caaggagggg agacaattgg gaccaacact    1380
agaatatcgc cctccctctg gtcctgaggg agaggaatcc tcctgggttt ccagatcctg    1440
taccagagag tgactctgag gttccgccct gctctctgac acaattaagg gataaaatct    1500
ctgacggaat gacggaaaga cgatccctcg aatactgatg actggttccc tttgacaccg    1560
gcagcagcct tgggaccgtg acttttcctc tcaggccttg ttctctgctt cacactcaat    1620
gtgtgtgggg gtctgagtcc agcacttctg agtccctcag cctccactca ggtcaggacc    1680
agaagtcgct gttccctcct cagggaatag aagattatcc caggtgcctg tgtccaggct    1740
ggtgtctggg ttctgtgctc tcttccccat cccgggtgtc ctgtccattc tcaagatggc    1800
cacatgcatg ctggtggagt gtcccatgac agatgcaaaa tgcctgaatt ttctgactct    1860
tcccgtcaga cccccccaag acacatatga cccaccaccc catctctgac catgaggcca    1920
ctctgagatg ctgggccctg ggcttctacc ctgcggagat cacactgacc tggcagcggg    1980
atggggagga ccagacccag gacacggagc ttgtggagac caggcctgca ggggatggaa    2040
ccttccagaa gtgggcagct gtggtggtac cttctggaga ggagcagaga tacacctgcc    2100
atgtgcagca tgagggtctg cccaagcccc tcaccctgag atggggtaag gagggagatg    2160
ggggtgtcat gtctcttagg gaaagcagga gcctctctgg agacctttag cagggtcagg    2220
gcccctcacc ttcccctctt ttcccagagc catcttccca gcccaccgtc cccatcgtgg    2280
gcatcattgc tggcctggtt ctccttggag ctgtgatcac tggagctgtg gtcgctgctg    2340
tgatgtggag gaggaacagc tcaggtggag aaggggtgaa gggtggggtc tgagatttct    2400
tgtctcactg agggttccaa gccccagcta gaaatgtgcc ctgtctcatt actgggaagc    2460
accatccaca atcatgggcc gacccagcct gggccctgtg tgccagcact tactcttttg    2520
taaagcacct gtgacaatga aggacagatt tatcaccttg attatggcgg tgatgggacc    2580
tgatcccagc agtcacaagt cacaggggaa ggtccctgac gacagatctc aggagggcga    2640
ttggtccagg gcccacatct gctttcttca tgtttcctga tcctgccctg ggtctgcagt    2700
cacacatttc tggaaacttc tctggggtcc aagactagga ggttcctcta ggaccttaag    2760
gccctggctc ctttctggta tctcacagga cattttcttc ccacagatag aaaaggaggg    2820
agctactctc aggctgcaag taagtatgaa ggaggctgat gcctgaggtc cttgggatat    2880
tgtgtttggg agcccatggg ggagctcaac caccccacaa ttcctcctct agccacatct    2940

tctgtgggat ctgaccaggt tctgtttttg ttctacccca ggcagtgaca gtgcccaggg 3000
ctctgatgtg tctctcacag cttgtaaagg tgagagcttg gagggcctga tgtgtgttgg 3060
gtgttgggcg gaacagtgga cacagctgtg ctatggggtt tctttgcatt ggatgtattg 3120
agcatgcgat gggctgttta aagtgtgacc cctcactgtg acagatatga agttgttcat 3180
gaattttttt tctatagtgt gagacagctg ccttgtgtgg gactgagagg caagagttgt 3240
tcctgccctt ccctttgtga cttgaagaac cctgactttg tttctgcaaa ggcacctgca 3300
tgtgtctgtg ttcatgtagg cataatgtga ggaggtgggg agaccacccc acccccatgt 3360
ccaccatgac cctcttccca cgctgacctg tgctccctcc ccaatcatct ttcctgttgc 3420
agagaggtgg ggctgaggtg tctccatctc tgtctcaact tcatggtgca ctgagctgta 3480
acttcttcct tccctattaa aa 3502

<210> 2
<211> 1098
<212> DNA
<213> Homo sapiens
<400> 2
ATG GCC GTC ATG GCG CCC CGA ACC CTC GTC CTG CTA CTC TCG GGG GCC 48
CTG GCC CTG ACC CAG ACC TGG GCA GGC TCC CAC TCC ATG AGG TAT TTC 96
TCC ACA TCC GTG TCC CGG CCC GGC CGC GGG GAG CCC CGC TTC ATC GCC 144
GTG GGC TAC GTG GAC GAC ACG CAG TTC GTG CGG TTC GAC AGC GAC GCC 192
GCG AGC CAG AGG ATG GAG CCG CGG GCG CCG TGG ATA GAG CAG GAG GGG 240
CCG GAG TAT TGG GAC GAG GAG ACA GGG AAA GTG AAG GCC CAC TCA CAG 288
ACT GAC CGA GAG AAC CTG CGG ATC GCG CTC CGC TAC TAC AAC CAG AGC 336
GAG GCC GGT TCT CAC ACC CTC CAG ATG ATG TTT GGC TGC GAC GTG GGG 384
TCG GAC GGG CGC TTC CTC CGC GGG TAC CAC CAG TAC GCC TAC GAC GGC 432
AAG GAT TAC ATC GCC CTG AAA GAG GAC CTG CGC TCT TGG ACC GCG GCG 480
GAC ATG GCG GCT CAG ATC ACC AAG CGC AAG TGG GAG GCG GCC CAT GTG 528
GCG GAG CAG CAG AGA GCC TAC CTG GAG GGC ACG TGC GTG GAC GGG CTC 576
CGC AGA TAC CTG GAG AAC GGG AAG GAG ACG CTG CAG CGC ACG GAC CCC    624
CCC AAG ACA CAT ATG ACC CAC CAC CCC ATC TCT GAC CAT GAG GCC ACT    672
CTG AGA TGC TGG GCC CTG GGC TTC TAC CCT GCG GAG ATC ACA CTG ACC    720
TGG CAG CGG GAT GGG GAG GAC CAG ACC CAG GAC ACG GAG CTT GTG GAG    768
ACC AGG CCT GCA GGG GAT GGA ACC TTC CAG AAG TGG GCA GCT GTG GTG    816
GTA CCT TCT GGA GAG GAG CAG AGA TAC ACC TGC CAT GTG CAG CAT GAG    864
GGT CTG CCC AAG CCC CTC ACC CTG AGA TGG GAG CCA TCT TCC CAG CCC    912
ACC GTC CCC ATC GTG GGC ATC ATT GCT GGC CTG GTT CTC CTT GGA GCT    960
GTG ATC ACT GGA GCT GTG GTC GCT GCT GTG ATG TGG AGG AGG AAC AGC   1008
TCA GAT AGA AAA GGA GGG AGC TAC TCT CAG GCT GCA AGC AGT GAC AGT   1056
GCC CAG GGC TCT GAT GTG TCT CTC ACA GCT TGT AAA GTG TGA           1098

<210>  3
<211>  24
<212>  DNA
<213>  Artificial Sequence
<220>
<223>  Description of Artificial Sequence: Primer-1
<400>  3
agggtcgggc gggtctcagc cact                                              24

<210>  4
<211>  29
<212>  DNA
<213>  Artificial Sequence
<220>
<223>  Description of Artificial Sequence: Primer-2
<400>  4
aaccgcacga actgcgtgtc gtccacgta                                         29

<210>  5
<211>  29
<212>  DNA
<213>  Artificial Sequence
<220>
<223>  Description of Artificial Sequence: Primer-3
<400>  5
tacgtggacg acacgcagtt cgtgcggtt                                          29

<210>  6
<211>  27
<212>  DNA
<213>  Artificial Sequence
<220>
<223>  Description of Artificial Sequence: Primer-4
<400>  6
tcaccggcct cgctctggtt gtagtag                                            27

<210>  7
<211>  25
<212>  DNA
<213>  Artificial Sequence
<220>
<223>  Description of Artificial Sequence: Primer-5
<400>  7
ggttgtagta gcggagcgcg atccg                                              25

<210>  8
<211>  21
<212>  DNA
<213>  Artificial Sequence
<220>
<223>  Description of Artificial Sequence: Primer-6
<400>  8
tagcggagcg cgatccgcag g                                                  21

<210>  9
<211>  20
<212>  DNA
<213>  Artificial Sequence
<220>
<223>  Description of Artificial Sequence: Primer-7
<400>  9
tagcggagcg cgatccgcag                                                    20

<210>  10
<211>  30
<212>  DNA
<213>  Artificial Sequence
<220>
<223>  Description of Artificial Sequence: Primer-8
<400>  10
cctcgctctg gttgtagtag cggagcgcta                                         30

<210>  11
<211>  25
<212>  DNA
<213>  Artificial Sequence
<220>
<223>  Description of Artificial Sequence: Primer-9
<400>  11
ggttgtagta gccgcgcagg gtccc                                              25

<210>  12
<211>  21
<212>  DNA
<213>  Artificial Sequence
<220>
<223>  Description of Artificial Sequence: Primer-10
<400>  12
tagccgcgca gggtccccag g                                                21

<210>  13
<211>  20
<212>  DNA
<213>  Artificial Sequence
<220>
<223>  Description of Artificial Sequence: Primer-11
<400>  13
tagccgcgca gggtccccag                                                  20

<210>  14
<211>  29
<212>  DNA
<213>  Artificial Sequence
<220>
<223>  Description of Artificial Sequence: Primer-12
<400>  14
ctcgctctgg ttgtagtagc cgcgcagtg                                        29

## Description

### TECHNICAL FIELD

The present invention relates to a method for determining whether an HLA type is an HLA-A*24 group or not.

Priority is claimed on Japanese Patent Application No. 2011-289336, filed on December 28, 2011, the content of which is incorporated herein by reference.

### BACKGROUND ART

HLA (Human Leukocyte Antigen) is a gene product of a gene group encoded in an MHC (Major Histocompatibility Complex) region present in the short arm portion of the sixth chromosome. The HLA has highly sufficient diversity, and it is important to improve the suitability of the HLA, in particular, for the work in the medical field of hematopoietic stem cell transplantation, blood transfusion, or the like. In this regard, there is a demand to carry out accurate HLA typing with as little effort as possible in the medical field.

HLA typing methods are roughly divided into a serological typing method and a DNA typing method. In the DNA typing method, it is possible to determine HLA types in detail, as compared to the serological typing method. Also with the DNA typing method, there sometimes occurs an ambiguity problem in that two or more kinds of alleles, which cannot be identified from the DNA typing results, exist and accordingly, the HLA types cannot be identified accurately.

For example, PTL 1 discloses a method for typing a subtype (type) of an HLA-A antigen at a genetic level. Specifically, the HLA-A type is typed by classifying HLA-A allele genes (alleles) into specific groups, carrying out PCR using a primer capable of specifically amplifying the HLA-A alleles for each group, and subjecting the PCR products thus obtained to an RFLP method, a PCR-RFLP method, an SSOP method, a PCR-SSOP method, a PCR-SSP method, or a PCR-SSCP method. It is expected that the ambiguity can be eliminated and the HLA-A types can thus be typed in detail by a multi-stage assay in the same manner as this method, but there remains a problem that a great deal of efforts are made.

Incidentally, the distribution of HLA types differ among races. According to the report from the HLA laboratory, a non-profit organization, the gene frequency of the HLA-A types in Japanese is the highest with the HLA-A*24:02 types, which account for 35.936%, as shown in Table 1, from the results of the typing of 21705 persons in 5538 Japanese families (as of December 19, 2011) (see, for example, NPL 1).

**[Table 1]**

| Allyl type | Gene frequency | Rank | Allele type | Gene frequency | Rank | Allele type | Gene frequency | Rank |
|---|---|---|---|---|---|---|---|---|
| A*24:02 | 35.936% | 1 | A*02:07 | 3.488% | 8 | A*24:04 | 0.014% | 27 |
| A*02:01 | 11.532% | 2 | A*26:03 | 2.414% | 9 | A*24:07 | 0.014% | 27 |
| A*02:06 | 9.247% | 3 | A*26:02 | 1.857% | 10 | A*24:03 | 0.009% | 31 |
| A*11:01 | 9.15% | 4 | A*24:20 | 0.71% | 11 | A*24:88 | 0.005% | 36 |
| A*31:01 | 8.685% | 5 | A*24:08 | 0.041% | 20 | | | |
| A*26:01 | 7.528% | 6 | A*32:01 | 0.032% | 22 | | | |
| A*33:03 | 7.192% | 7 | A*24:25 | 0.018% | 25 | | | |

### PRIOR ART DOCUMENTS

### PATENT DOCUMENTS

PTL 1: Japanese Unexamined Patent Application, First Publication No. H11-216000

### Non-Patent Literature

NPL 1: "Allele frequency search 1 seat/A search" [on line], Public interest incorporated foundation, HLA Laboratory, [search on December 19, 2013], Internet <URL: http://www.hla.or.jp/haplo/haplo-search.php?type=aril&loci=A&lang=ja>

### SUMMARY OF INVENTION

### PROBLEMS TO BE SOLVED BY THE INVENTION

It is an object of the present invention to provide a method for determining the typing of an HLA-A*24 group with less ambiguity in a simple manner.

### MEANS FOR SOLVING THE PROBLEMS

The present inventors have made extensive studies to solve the above-described problems, and as a result, they have found that the allele of an HLA-A*24 group can be determined with high precision by sufficiently reducing the ambiguity through a very small number of steps, on the basis of the results of the typing of (a) a 211st base and (b) a 395th base, in which A (adenine) in the initiation codon for the HLA gene is defined as the 1 st base, thereby completing the present invention.

That is, the present invention provides:
(1) a method for determining an HLA-A*24 group,which includes determining the HLA-A*24 group on the basis of the results of the typing of (a) a 211st base and (b) a 395th base in a genomic DNA of a subject, in which A (adenine) in the initiation codon for the HLA gene is defined as the 1st base in the genomic DNA of the test subject;
(2) the method for determining an HLA-A*24 group according to (1), whether the HLA-A*24 group is determined, further on the basis of the results of the typing of (c) at least one base selected from a 437th base, a 441st base, a 443rd base, a 444th base, a 447th base, and a 449th base;
(3) the method for determining an HLA-A*24 group according to (2), wherein it is determined whether or not the HLA type of the test subject is HLA-A*24:02;
(4) the method for determining an HLA-A*24 group according to (3), wherein when the 211st base is C (cytosine), the 395th base is G (guanine), the 437th base is C, the 441st base is T (thymine), the 443rd base is G, the 444th base is C, the 447th base is T, and the 449th base is C in at least one allele, the HLA type of the test subject is determined to be HLA-A*24:02;
(5) the method for determining an HLA-A*24 group according to any one of (1) to (4), wherein the typing of the bases of (a) and (b) is carried out by a sequence analysis method or a single-base detection method;
(6) the method for determining an HLA-A*24 group according to any one of (2) to (5), wherein the typing of the bases of (c) is carried out by a sequence analysis method or a single-base detection method;
(7) the method for determining an HLA-A*24 group according to any one of (2) to (5), wherein when an elongation product is obtained by an elongation reaction by a polymerase, using a primer containing a base sequence represented by SEQ NO: 7, the bases of (c) are typed as follows; the 437th base is C, the 441st base is T, the 443rd base is G, the 444th base is C, the 447th base is T, and the 449th base is C; and
(8) a kit for determining HLA-A*24:02, including a primer for determining HLA-A*24:02 containing a base sequence represented by SEQ NO: 7.

### Effects of the Invention

By the method for determining an HLA-A*24 group of the present invention, the typing of the HLA-A*24 group can be determined with high precision in a simpler manner. The method for determining an HLA-A*24 group of the present invention can determine the alleles of an HLA-A*24 group having high gene frequency, particularly in the Japanese, and therefore, it is particularly suitable for determination when the test subject is Japanese.

### BRIEF DESCRIPTION OF THE DRAWINGS

FIG. 1A is a view showing the -300th to 600th bases, in which A (adenine) in the initiation codon for the HLA gene is defined as the 1 st base, in the base sequence of the genomic DNA of the HLA gene;
FIG. 1B is a view showing the 601st to 1500th bases, in which A (adenine) in the initiation codon for the HLA gene is defined as the 1 st base, in the base sequence of the genomic DNA of the HLA gene;
FIG. 1C is a view showing the 1501st to 2400th bases, in which A (adenine) in the initiation codon for the HLA gene is defined as the 1 st base, in the base sequence of the genomic DNA of the HLA gene;
FIG. 1D is a view showing the 2401st to 3202nd bases, in which A (adenine) in the initiation codon for the HLA gene is defined as the 1 st base, in the base sequence of the genomic DNA of the HLA gene;
FIG. 2 is a view showing the base sequence of each allele at the 211st base ("(a)" in the figure) and its vicinity, the 395th base ("(b)" in the figure) and its vicinity, and the 437th to 449th bases ("(c)" in the figure) and its vicinity in the second exon of the genomic DNA of the HLA gene.
FIG. 3 is a view schematically showing an embodiment of a method for typing the bases of (a) to (c); and
FIG. 4 is a view schematically showing an embodiment of a method for typing the bases of (a) to (c).

### EMBODIMENTS FOR CARRYING OUT THE INVENTION

In the present invention and the present specification, the expression "typing a base" is meant to determine the base among A (adenine), G (guanine), C (cytosine), and T (thymine).

In the method for determining an HLA-A*24 group of the present invention (hereinafter sometimes referred to as "the determination method of the present invention"), the allele of the HLA-A*24 group is determined, wherein the allele of an HLA-A*24 group is determined on the basis of the results of the typing of (a) a 211st base and (b) a 395th base, in which A (adenine) in the initiation codon for the HLA gene is defined as the 1st base, in the genomic DNA of the test subject.

Furthermore, the determination method of the present invention may be further based on the results of the typing of at least one selected from the group consisting of (c) the 437th base, the 441st base, the 443rd base, the 444th base, the 447th base, and the 449th base, in addition to the results of the typing of the bases of (a) and (b). Further, since the 437th base, the 441st base, the 443rd base, the 444th base, the 447th base, and the 449th base are chained, at least one base of the 437th base, the 441st base, the 443rd base, the 444th base, the 447th base, and the 449th base may be typed. Further, the SNP (Single Nucleotide Polymorphism) characterizing the HLA-A*24:02 is not limited to the above, but a base distinct from an HLA-A group that is present with an allele frequency of 0.001% or more is preferred.

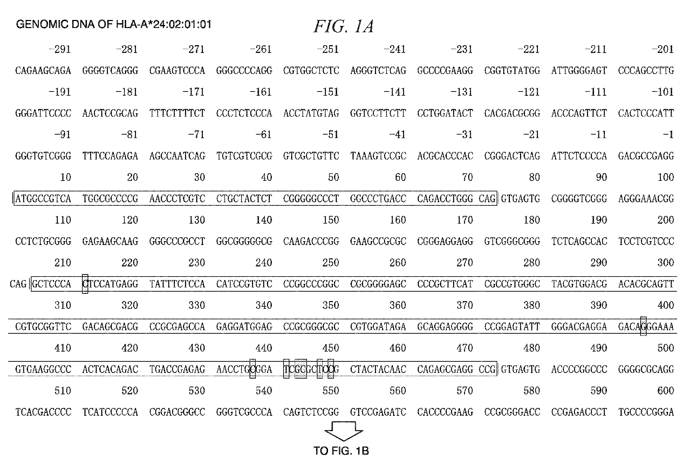

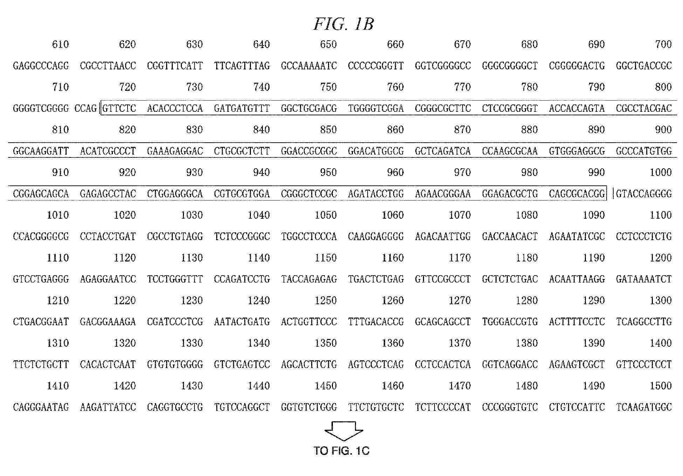

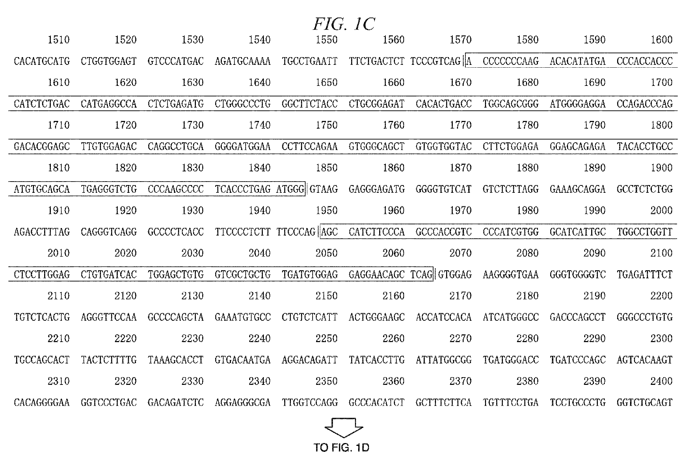

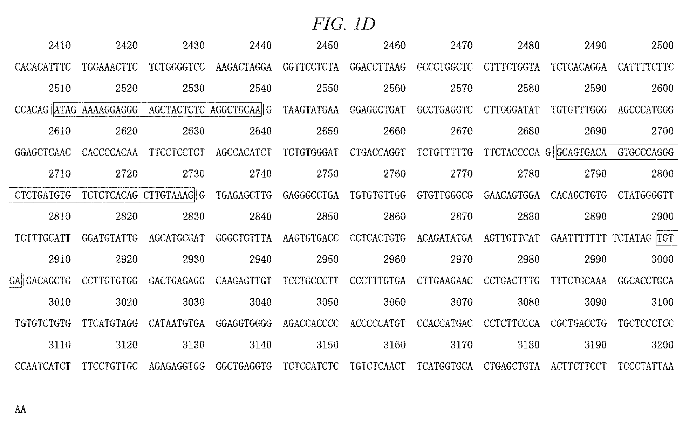

FIG. 1 shows the base sequence (SEQ NO: 1) of the genomic DNA of the HLA gene. In FIGS. 1A to 1D, the region surrounded in a square is a coding region and the number of each base is assigned on the basis that A (adenine) in the initiation codon for the HLA gene is defined as the 1st base. The 211st base (C), the 395th base (G), the 437th base (C), the 441st base (T), the 443rd base (G), the 444th base (C), the 447th base (T), and the 449th base (C) are shaded. Further, the amino acid sequence corresponding to the base sequence of the coding region of the HLA gene is shown in SEQ NO: 2.

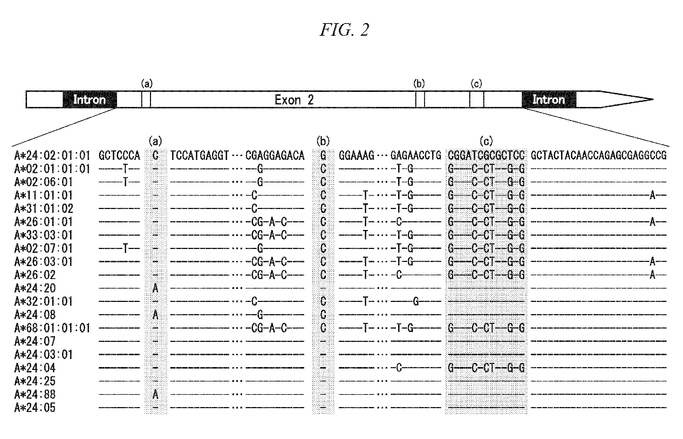

These bases are all present in the second exons. In FIG. 2, the base sequence of each allele of the 211st base ("(a)" in the figure) and its vicinity, the 395th base ("(b)" in the figure) and its vicinity, and the 437th to 449th 6bases ("(c)" in the figure) and its vicinity in the second exon is shown. In the bases of each allele in the figures, "-" represents a base that is analogous to HLA-A*24:02.

The gene frequency of each allele in the Japanese and the bases of (a) to (c) above are shown in Table 2. Further, as the bases of (c), the 437th base was used. In addition, in Table 2, "others" means all the HLA-A types other than the HLA-A types specifically shown in Table 2.

**[Table 2]**

| Gene frequency (%) | Allele type | (a) | (b) | (c) |
|---|---|---|---|---|
| 35.936 | A*24:02 | C | G | C |
| 0.71 | A*24:20 | A | G | C |
| 0.032 | A*32:01 | C | C | C |
| 0.041 | A*24:08 | A | C | C |
| 0.014 | A*24:07 | C | G | C |
| 0.009 | A*24:03 | C | G | C |
| 0.014 | A*24:04 | C | G | G |
| 0.018 | A*24:25 | C | G | C |
| 0.005 | A*24:88 | A | G | C |
| 63.22 | Others | C | C | G |

When the 211st base ((a)) is A or C, the 395th base ((b)) is G or C, and neither the 211st base nor the 395th base is C in at least one allele, the HLA type of the test subject is determined to be an HLA-A*24 group.

The determination results in the case of typing the bases of (a) and (b) in the HLA gene included in the genomic DNA of the test subject, and determining the HLA-A type of the test subject on the basis of the results of the typing thus obtained, and the actual genetic types are shown in Table 3, together with the gene frequency. In Table 3, the column of "A" shows the allele types (HLA-A types) and the "others" have the same meanings as in Table 2. Further, the "+" in the column of "Type" represents a genomic DNA including the allele of an HLA-A*24 group, and the "-" represents a genomic DNA including no allele of an HLA-A*24 group. Further, when at least one A or C exists in the column of "(a)" and at least one G or C exists in the column of "(b)" (provided that in the case where only C exists in the column of "(a)" (both of the alleles are C), and only C exists in the column of "(b)" is excluded), the genomic DNA is determined to include the allele of the HLA-A*24 group (that is, "+" is denoted in the column of "Determination"), and in the other cases, the genomic DNA is determined to include no allele of the HLA-A*24 group (that is, "-" is denoted in the column of "Determination"). Further, in the column of "Errata", "○" denotes a case where the columns of "Type" and "Determination" are consistent with each other, whereas "x" denotes a case where the columns of "Type" and "Determination" are not consistent with each other. The "gene frequency" is defined as a product obtained by multiplying the gene frequencies of the respective alleles (for example, in the case of the homotype of A*24:02, 35.936%x35.936%=12.91%). As shown in Table 3, in the determination method of the present invention, it can be determined whether the allele of the HLA-A*24 group is included or not in the genomic DNA of the test subject, on the basis of only the results of the typing of the bases of (a) and (b), with a higher precision.

**[Table 3]**

| A | A | (a) | (b) | Type | Determination | Errata | Frequency (%) |
|---|---|---|---|---|---|---|---|
| A*24:02 | A*24:02 | C/C | G/G | + | + | ○ | 12.91 |
| A*24:02 | A*24:20 | A/C | G/G | + | + | ○ | 0.51 |
| A*24:02 | A*32:01 | C/C | C/G | + | + | ○ | 0.02 |
| A*24:02 | A*24:08 | A/C | C/G | + | + | ○ | 0.03 |
| A*24:02 | A*24:07/03/25 | C/C | G/G | + | + | ○ | 0.03 |
| A*24:02 | A*24:04 | C/C | G/G | + | + | ○ | 0.01 |
| A*24:02 | A*24:88 | A/C | G/G | + | + | ○ | 0.00 |
| A*24:02 | Others | C/C | C/G | + | + | ○ | 45.44 |
| A*24:20 | A*24:20 | A/A | G/G | + | + | ○ | 0.01 |
| A*24:20 | A*32:01 | A/C | C/G | + | + | ○ | 0.00 |
| A*24:20 | A*24:08 | A/A | C/G | + | + | ○ | 0.00 |
| A*24:20 | A*24:07/03/25 | A/C | G/G | + | + | ○ | 0.00 |
| A*24:20 | A*24:04 | A/C | G/G | + | + | ○ | 0.00 |
| A*24:20 | A*24:88 | A/A | G/G | + | + | ○ | 0.00 |
| A*24:20 | Others | A/C | C/G | + | + | ○ | 0.90 |
| A*32:01 | A*32:01 | C/C | C/C | - | - | ○ | 0.00 |
| A*32:01 | A*24:08 | A/C | C/C | + | + | ○ | 0.00 |
| A*32:01 | A*24:07/03/25 | C/C | C/G | + | + | ○ | 0.00 |
| A*32:01 | A*24:04 | C/C | C/G | + | + | ○ | 0.00 |
| A*32:01 | A*24:88 | A/C | C/G | + | + | ○ | 0.00 |
| A*32:01 | Others | C/C | C/C | - | - | ○ | 0.04 |
| A*24:08 | A*24:08 | A/A | C/C | + | + | ○ | 0.00 |
| A*24:08 | A*24:07/03/25 | A/C | C/G | + | + | ○ | 0.00 |
| A*24:08 | A*24:04 | A/C | C/G | + | + | ○ | 0.00 |
| A*24:08 | A*24:88 | A/A | C/G | + | + | ○ | 0.00 |
| A*24:08 | Others | A/C | C/C | + | + | ○ | 0.05 |
| A*24:07/03/25 | A*24:07/03/25 | C/C | G/G | + | + | ○ | 0.00 |
| A*24:07/03/25 | A*24:04 | C/C | G/G | + | + | ○ | 0.00 |
| A*24:07/03/25 | A*24:88 | A/C | G/G | + | + | ○ | 0.00 |
| A*24:07/03/25 | Others | C/C | C/G | + | + | ○ | 0.05 |
| A*24:04 | A*24:04 | C/C | G/G | + | + | ○ | 0.00 |
| A*24:04 | A*24:88 | A/C | G/G | + | + | ○ | 0.00 |
| A*24:04 | Others | C/C | C/G | + | + | ○ | 0.02 |
| A*24:88 | A*24:88 | A/A | G/G | + | + | ○ | 0.00 |
| A*24:88 | Others | A/C | C/G | + | + | ○ | 0.01 |
| Others | Others | C/C | C/C | - | - | ○ | 39.97 |

Moreover, as shown in Table 2, in the HLA-A*24 group except for the HLA-A*24:04, the bases of (c) are different from those of the alleles other than the HLA-A*24 group. Accordingly, the results of the typing of the bases of (c) may be used, in addition to the results of the typing of the bases of (a) and (b).

For determining whether the allele of the HLA-A*24 group is included in the genomic DNA of the test subject, the use of only the results of the typing of the bases of (a) and (b) is sufficient, but the HLA type can be determined in more detail by using them in combination with the results of the typing of the other bases. For example, the HLA-A*24:02 in the HLA-A*24 group can be determined in more detail by using the results of the typing of the bases of (c) in addition to the results of the typing of the bases of (a) and (b). Since the HLA-A*24:02 has the highest gene frequency in the Japanese, the embodiment is particularly useful for clinical tests, in which the test subjects are Japanese.

Specifically, when these bases are all the same as HLA-A*24:02, that is, the 211st base is C, the 395th base is G, the 437th base is C, the 441st base is T, the 443rd base is G, the 444th base is C, the 447th base is T, and the 449th base is C in at least one allele, the HLA type of the test subject is determined to be HLA-A*24:02. Further, when the 437th base is C, then the 441st base is T, the 443rd base is G, the 444th base is C, the 447th base is T, and the 449th base is C in any case. That is, in the determination method of the present invention, it can be determined whether HLA-A*24:02 is included in a genomic DNA of a test subject, on the basis of the results of the typing of three bases.

As shown in Table 2, the results of the typing of the bases of (a) to (c) of the HLA-A*24:07, the HLA-A*24:03, and the HLA-A*24:25 are the same as those of the HLA-A*24:02, but the alleles of the other HLA-A types are different from those of the HLA-A*24:02.

That is, from the results of the typing of the bases of (a) to (c), it is impossible to identify the HLA-A*24:02 from the HLA-A*24:07, the HLA-A*24:03, and the HLA-A*24:25, but it is possible to determine the HLA-A*24:02 from the alleles of the other types.

The bases of (a) to (c) in the HLA gene included in the genomic DNA of the test subject is typed, then the determination results in the case of determining the HLA-A type of the test subject based on the typing results thus obtained and the determination criteria shown in Table 4 and the actual genetic types are shown in Table 5, together with the gene frequency. In Table 5, the column of "A" shows the allele types (HLA-A types) and the "others" has the same meaning as in Table 2. Further, in the column of "Type", "+" denotes a genomic DNA including the HLA-A*24:02 and "-" denotes a genomic DNA not including the HLA-A*24:02. In Tables 4 and 5, in the column of "Determination", "+" denotes that the genomic DNA of the test subject is determined to include the HLA-A*24:02 and "-" denotes that the genomic DNA of the test subject is determined to include no HLA-A*24:02. That is, in the case where there is at least one C in the column of "(a)", there is at least one G in the column of "(b)", and there is at least one C in the column of "(c)", and also there is no G in the column of "(c)" if there is A in the column of "(a)", the genomic DNA is determined to include the HLA-A*24:02 (that is, in the column of "Determination", "+" is denoted). In the case where there is no C in the column of "(a)"; in the case where there is no G in the column of "(b)"; or in the case where there is no C in the column of "(c)"; and also or in the case where there is at least one A in the column of "(a)", there is at least one G in the column of "(b)", and there is at least one G in the column of "(c)", the genomic DNA is determined not to include the HLA-A*24:02 (that is, "-" denotes such the cases in the column of "Determination"). In addition, in the column of "Errata", "○" denotes a case where the columns of "Type" and "Determination" are consistent with each other, whereas "x" denotes a case where the columns of "Type" and "Determination" are not consistent with each other.

**[Table 4]**

| (a) | (b) | (c) | Determination |
|---|---|---|---|
| C/C | G/G | C/C | + |
| A/C | G/G | C/C | + |
| C/C | C/G | C/C | + |
| A/C | C/G | C/C | + |
| C/C | G/G | C/G | + |
| C/C | C/G | C/G | + |
| | | | |
| A/A | G/G | C/C | - |
| A/A | C/G | C/C | - |
| A/A | C/C | C/C | - |
| A/C | G/G | C/G | - |
| A/C | C/G | C/G | - |
| A/C | C/C | C/G | - |
| A/C | C/C | C/C | - |
| C/C | G/G | G/G | - |
| C/C | C/G | G/G | - |
| C/C | C/C | G/G | - |
| C/C | C/C | C/G | - |
| C/C | C/C | C/C | - |

**[Table 5]**

| A | A | (a) | (b) | (c) | Type | Determination | Errata | Frequency (%) |
|---|---|---|---|---|---|---|---|---|
| A*24:02 | A*24:02 | C/C | G/G | C/C | + | + | ○ | 12.91 |
| A*24:02 | A*24:20 | A/C | G/G | C/C | + | + | ○ | 0.51 |
| A*24:02 | A*32:01 | C/C | C/G | C/C | + | + | ○ | 0.02 |
| A*24:02 | A*24:08 | A/C | C/G | C/C | + | + | ○ | 0.03 |
| A*24:02 | A*24:07/03/25 | C/C | G/G | C/C | + | + | ○ | 0.03 |
| A*24:02 | A*24:04 | C/C | G/G | C/G | + | + | ○ | 0.01 |
| A*24:02 | A*24:88 | A/C | G/G | C/C | + | + | ○ | 0.00 |
| A*24:02 | Others | C/C | C/G | C/G | + | + | ○ | 45.44 |
| A*24:20 | A*24:20 | A/A | G/G/ | C/C | - | - | ○ | 0.01 |
| A*24:20 | A*32:01 | A/C | C/G | C/C | - | + | × | 0.00 |
| A*24:20 | A*24:08 | A/A | C/G | C/C | - | - | ○ | 0.00 |
| A*24:20 | A*24:07/03/25 | A/C | G/G | C/C | - | + | × | 0.00 |
| A*24:20 | A*24:04 | A/C | G/G | C/G | - | - | ○ | 0.00 |
| A*24:20 | A*24:88 | A/A | G/G | C/C | - | - | ○ | 0.00 |
| A*24:20 | Others | A/C | C/G | C/G | - | - | ○ | 0.90 |
| A*32:01 | A*32:01 | C/C | C/C | C/C | - | - | ○ | 0.00 |
| A*32:01 | A*24:08 | A/C | C/C | C/C | - | - | ○ | 0.00 |
| A*32:01 | A*24:07/03/25 | C/C | C/G | C/C | - | + | × | 0.00 |
| A*32:01 | A*24:04 | C/C | C/G | C/G | - | + | × | 0.00 |
| A*32:01 | A*24:88 | A/C | C/G | C/C | - | + | × | 0.00 |
| A*32:01 | Others | C/C | C/C | C/G | - | - | ○ | 0.04 |
| A*24:08 | A*24:08 | A/A | C/C | C/C | - | - | ○ | 0.00 |
| A*24:08 | A*24:07/03/25 | A/C | C/G | C/C | - | + | × | 0.00 |
| A*24:08 | A*24:04 | A/C | C/G | C/G | - | - | ○ | 0.00 |
| A*24:08 | A*24:88 | A/A | C/G | C/C | - | - | ○ | 0.00 |
| A*24:08 | Others | A/C | C/C | C/G | - | - | ○ | 0.05 |
| A*24:07/03/25 | A*24:07/03/25 | C/C | G/G | C/C | - | + | × | 0.00 |
| A*24:07/03/25 | A*24:04 | C/C | G/G | C/G | - | + | × | 0.00 |
| A*24:07/03/25 | A*24:88 | A/C | G/G | C/C | - | + | × | 0.00 |
| A*24:07/03/25 | Others | C/C | C/G | C/G | - | + | × | 0.05 |
| A*24:04 | A*24:04 | C/C | G/G | G/G | - | - | ○ | 0.00 |
| A*24:04 | A*24:88 | A/C | G/G | C/G | - | - | ○ | 0.00 |
| A*24:04 | Others | C/C | C/G | G/G | - | - | ○ | 0.02 |
| A*24:88 | A*24:88 | A/A | G/G | C/C | - | - | ○ | 0.00 |
| A*24:88 | Others | A/C | C/G | C/G | - | - | ○ | 0.01 |
| Others | Others | C/C | C/C | G/G | - | - | ○ | 39.97 |

From the results of the typing of the (a) to (c) bases, when the test subject has a genomic DNA represented by x in the column of "Errata" in Table 5, it is mistakenly determined that the test subject has HLA-A*24:02 even though the genomic DNA does not actually include the HLA-A*24:02 (false-positive). However, for the Japanese, any of the gene frequency of the genomic DNA that is false-positive is extremely low, and accordingly, the proportion of the test subject determined to be false-positive is suppressed to only 0.05%. For example, when the method for determining whether or not the HLA type of the test subject is HLA-A*24:02, using the results of the typing of the bases of (c), in addition to (a) and (b) (hereinafter sometimes referred to as the "method for determining HLA-A*24:02 of the present invention") among the determination methods of the present invention is carried out for 1,000,000 Japanese persons, none is determined to be false-negative and 530 persons are determined to be false-positives, of which the number is critically low in theory, as shown in Table 6, despite determining based on the results of the typing of only three bases.

**[Table 6]**

| | At a test for 1,000,000 persons (persons) | Frequency (%) |
|---|---|---|
| Positive | 589,573 | 59 |
| Negative | 409,877 | 41 |
| False-negative | 0 | 0.00 |
| False-positive | 530 | 0.05 |

Most of the alleles of HLA-A pertain to a combination of a plurality of polymorphisms, and therefore, when only a part of a site (polymorphic site) different from the other allele among the alleles is typed, it is difficult to determine the plurality of alleles from each other. When a test for the purpose of determining a specific allele is conducted, an allele, which is not a desired allele in fact, is mistakenly determined to be the desired allele (false-positive), if accompanied by an occurrence of ambiguity. The problems related to the false-positive determination can be solved by eliminating any ambiguity, but a large number of bases to be typed is required in order to eliminate any ambiguity, leading to an excessive increase in the test cost.

That is, in order to apply the method for determining HLA to medical fields of a clinical test or the like, it becomes difficult to achieve a balance between the test accuracy and cost.

In the method for determining HLA-A*24:02 of the present invention, the bases to be used for the typing are identified by considering the gene frequency to minimize the probability of false-positives due to ambiguity as well as to minimize the number of bases to be typed in order to determine HLA-A*24:02. That is, since the method for determining HLA-A*24:02 of the present invention has a high determination accuracy of HLA-A*24:02 and is excellent in the test cost, it is suitable for a clinical test or the like.

If the determination is carried out based on only the results of the typing of the bases of (a) and (b), as shown in Table 7, the test subjects that are "HLA-A*24:20 and others (alleles other than the alleles described in Table 2) having a high gene frequency" are mistakenly determined to include HLA-A*24:02. As a result, the probability of the false-positives is increased to 0.97%, and thus, when tested for 1,000,000 Japanese persons, 9,749 persons are determined to be false-positive in theory. From this calculation, it is apparent that the determination accuracy of the method for determining HLA-A*24:02 of the present invention is high.

**[Table 7]**

| A | A | (a) | (b) | Type | Determination | Errata | Frequency (%) |
|---|---|---|---|---|---|---|---|
| A*24:02 | A*24:02 | C/C | G/G | + | + | ○ | 12.91 |
| A*24:02 | A*24:20 | A/C | G/G | + | + | ○ | 0.51 |
| A*24:02 | A*32:01 | C/C | C/G | + | + | ○ | 0.02 |
| A*24:02 | A*24:08 | A/C | C/G | + | + | ○ | 0.03 |
| A*24:02 | A*24:07/03/25 | C/C | G/G | + | + | ○ | 0.03 |
| A*24:02 | A*24:04 | C/C | G/G | + | + | ○ | 0.01 |
| A*24:02 | A*24:88 | A/C | G/G | + | + | ○ | 0.00 |
| A*24:02 | Others | C/C | C/G | + | + | ○ | 45.44 |
| A*24:20 | A*24:20 | A/A | G/G | - | - | ○ | 0.01 |
| A*24:20 | A*32:01 | A/C | C/G | - | + | × | 0.00 |
| A*24:20 | A*24:08 | A/A | C/G | - | - | ○ | 0.00 |
| A*24:20 | A*24:07/03/25 | A/C | G/G | - | + | × | 0.00 |
| A*24:20 | A*24:04 | A/C | G/G | - | + | × | 0.00 |
| A*24:20 | A*24:88 | A/A | G/G | - | - | ○ | 0.00 |
| A*24:20 | Others | A/C | C/G | - | + | × | 0.90 |
| A*32:01 | A*32:01 | C/C | C/C | - | - | ○ | 0.00 |
| A*32:01 | A*24:08 | A/C | C/C | - | - | ○ | 0.00 |
| A*32:01 | A*24:07/03/25 | C/C | C/G | - | + | × | 0.00 |
| A*32:01 | A*24:04 | C/C | C/G | - | + | × | 0.00 |
| A*32:01 | A*24:88 | A/C | C/G | - | + | × | 0.00 |
| A*32:01 | Others | C/C | C/C | - | - | ○ | 0.04 |
| A*24:08 | A*24:08 | A/A | C/C | - | - | ○ | 0.00 |
| A*24:08 | A*24:07/03/25 | A/C | C/G | - | + | × | 0.00 |
| A*24:08 | A*24:04 | A/C | C/G | - | + | × | 0.00 |
| A*24:08 | A*24:88 | A/A | C/G | - | - | ○ | 0.00 |
| A*24:08 | Others | A/C | C/C | - | - | ○ | 0.05 |
| A*24:07/03/25 | A*24:07/03/25 | C/C | G/G | - | + | × | 0.00 |
| A*24:07/03/25 | A*24:04 | C/C | G/G | - | + | × | 0.00 |
| A*24:07/03/25 | A*24:88 | A/C | G/G | - | + | × | 0.00 |
| A*24:07/03/25 | Others | C/C | C/G | - | + | × | 0.05 |
| A*24:04 | A*24:04 | C/C | G/G | - | + | × | 0.00 |
| A*24:04 | A*24:88 | A/C | G/G | - | + | × | 0.00 |
| A*24:04 | Others | C/C | C/G | - | + | × | 0.02 |
| A*24:88 | A*24:88 | A/A | G/G | - | - | ○ | 0.00 |
| A*24:88 | Others | A/C | C/G | - | + | × | 0.01 |
| Others | Others | C/C | C/C | - | - | ○ | 39.97 |

A nucleic acid sample provided for the determination method of the present invention may be one including nucleic acids having the base sequence of a region including the bases of (a) and (b) of the genomic DNA of the test subject (the bases of (a) to (c) of the genomic DNA of the test subject in the case of the method for determining HLA-A*24:02 of the present invention) reflected therein, and it may include either a genomic DNA or mRNA derived from the test subject.

For the typing of each base in an mRNA, the mRNA may be directly typed or may be typed using a cDNA synthesized by reverse transcription from the mRNA. The genomic DNA or mRNA derived from the test subject may be a nucleic acid that is extracted and purified from a biological sample taken from the test subject or may be a nucleic acid before purification. In addition, an amplification product obtained by amplifying a region including the bases of (a) and (b) or a region including the bases of (a) to (c) in the genomic DNA or mRNA derived from the test subject by PCR or the like may be used.

In the determination method of the present invention, the method for typing the bases of (a) to (c) are not particularly limited and may be a sequence analysis method which is based on a Sanger or a single-base detection method. As the single-base detection method, various known methods used in the detection of mutation of a gene, detection of polymorphism, or the like, for example, a PCR-SSO (sequence specific oligonucleotide) method, a PCR-SSP (sequence specific primer) method, or a modified method thereof may be used. The PCR-SSO method is a typing method according to the presence or absence for formation of an aggregate with the probe, using a probe that hybridizes specifically with a specific allele (allele-specific probe). The PCR-SSP method is a method for typing according to the presence or absence for a PCR product when carrying out PCR using a primer that hybridizes specifically with a specific allele (allele-specific probe). Examples of the modified method thereof include an Invader method, a TaqMan probe method, a QProbe method, and a Scorpion-ARMS method. A detection method using fluorescence is preferred due to its superior detection sensitivity, and a method using a probe or primer that recognizes a specific allele, an Invader method, a TaqMan probe method, a QProbe method, and a Scorpion-ARMS method is preferred due to its superior detection sensitivity and accuracy. For example, the probe or primer used for the typing of the bases of (a) to (c) can be synthesized by designing by an ordinary method, for example, based on the base sequence 1 or 2.

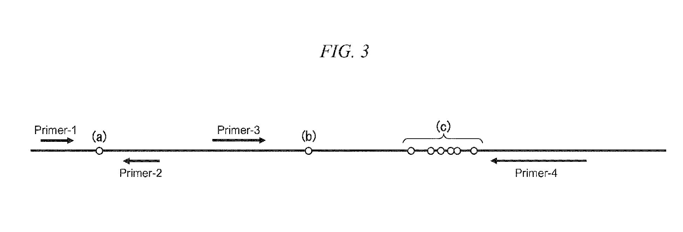

In the case of the typing by a sequence analysis method, the bases of (a) can be typed by carrying out PCR with a PCR primer (a primer-1 and a primer-2 in FIG. 3) for the amplification of a region having the bases of (a) interposed therein and analyzing the base sequence of the obtained amplification product, using a genomic DNA or mRNA derived from a test subject (hereinafter sometimes simply referred to as a "genomic DNA") as a template, for example, as shown in Figure 3. Similarly, the bases of (b) and (c) can be typed by carrying out PCR with a PCR primer (a primer-3 and a primer-4 in FIG. 3) for the amplification of a region having the bases of (b) and (c) interposed therein and analyzing the base sequence of the obtained amplification product. The six bases of (c) in FIG. 3 represent the 437th base, the 441st base, the 443rd base, the 444th base, the 447th base, and the 449th base from the upstream.

The primers-1 to -4 are not particularly limited as long as they are primers capable of amplifying a desired region including each of the bases of (a) to (c). Examples thereof include a primer including the base sequence shown in Table 8. In the case of typing only the bases of (a) and (b) by a sequence analysis method, PCR may be carried out using a PCR primer for the amplification of a region including the bases of (b), not including the bases of (c), and the base sequence of the amplification product thus obtained may be analyzed.

**[Table 8]**

| Primer | Base sequence (5' → 3') | SEQ NO: |
|---|---|---|
| Primer-1 | AGGGTCGGGCGGGTCTCAGCCACT | 3 |
| Primer-2 | AACCGCACGAACTGCGTGTCGTCCACGTA | 4 |
| Primer-3 | TACGTGGACGACACGCAGTTCGTGCGGTT | 5 |
| Primer-4 | TCACCGGCCTCGCTCTGGTTGTAGTAG | 6 |

Moreover, the typing of the bases may be carried out by a combination of a sequence analysis and a single-base detection method. For example, any of the bases of (a) to (c) may be subjected to PCR using a primer that hybridizes specifically with an allele of an HLA-A*24 group and typed according to the presence or absence of PCR products, and the remaining bases may be typed by a sequence analysis method.

Furthermore, the "primer that hybridizes specifically with a specific allele" is not limited to a primer having a base sequence fully complementary to a targeted allele, and it may be any primer that hybridizes preferentially with a targeted allele rather than other alleles or may include a mismatch base of one to several bases in a region that hybridizes with a targeted allele.

In the method for determining HLA-A*24:02 of the present invention, for example, any one of the bases of (a) to (c) may be subjected to PCR using a primer that hybridizes specifically with HLA-A*24:02 (HLA-A*24:02-specific primer) and typed according to the presence or absence of PCR products, and the remaining bases may be typed by a sequence analysis method.

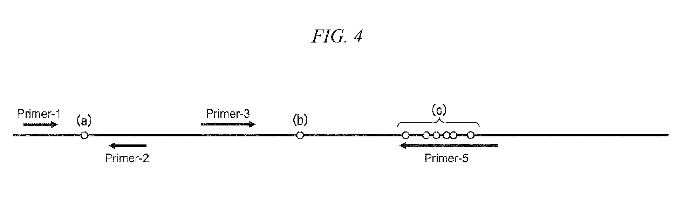

For example, as shown in FIG. 4, an elongation reaction using a polymerase such as PCR may be carried out, using a primer that hybridizes specifically with a region including the six bases of (c) (primer-5 in FIG. 4) and a primer that recognizes a region upstream of the bases of (b)(primer-3 in FIG. 4), with a genomic DNA derived from a test subject as a template. Further, the bases of (b) and (c) may be typed by investigating the presence or absence of PCR products and analyzing the base sequence of the PCR product. The typing of the bases of (a) may be carried out in the same manner as in FIG. 3.

In the case of the typing method shown in Fig. 3, PCR products are obtained, using many alleles of an HLA gene included in the genomic DNA as a template. Therefore, when the HLA gene is a heterotype, the bases of (a) to (c) may be sometimes typed as two types of bases, depending on typing methods, or may be mistakenly determined even with the use of the results of the typing of (a) to (c). To the contrary, when a region including the six bases of (c) is identified with a primer and the bases of (b) are typed by a sequence analysis method or the like, as in the typing method shown in FIG. 4, the results from the combination of the bases of (b) and the bases of (c) are obtained. That is, when the amplification is carried out using a primer which is specific to a group including HLA-A*24:02, the typing of the HLA-A*24:02 can be determined with high precision in a simpler manner than the typing method of FIG. 3, only by the determination of the bases of (a) and (b), even though the genomic DNA is a heterotype including HLA-A*24:02.

The primer-5 is not particularly limited as long as it is a primer capable of hybridizing specifically to a region including at least one of the six bases of (c). Examples of the primer to be used as the HLA-A*24:02-specific primer include a primer-5 including the base sequence shown in Table 9. The primers-6 to -8 shown in Table 9 may also be used as the HLA-A*24:02-specific primer for typing the bases of (c) in the same manner as the primer-5.

**[Table 9]**

| Primer | Base sequence (5' → 3') | SEQ NO: |
|---|---|---|
| Primer-5 | GGTTGTAGTAGCGGAGCGCGATCCG | 7 |
| Primer-6 | TAGCGGAGCGCGATCCGCAGG | 8 |
| Primer-7 | TAGCGGAGCGCGATCCGCAG | 9 |
| Primer-8 | CCTCGCTCTGGTTGTAGTAGCGGAGCGCTA | 10 |

When the typing is carried out depending on the presence or absence of PCR products as shown in FIG. 4 and PCR products are failed to be obtained, it is hard to judge whether the cause of the failure was that the genomic DNA as a template did not include an HLA-A*24 group or that the reaction system itself had a problem. Therefore, it is preferable to carry out PCR with the genomic DNA as a template, using a primer designed to ensure that PCR products are obtained when the genomic DNA does not include the HLA-A*24 group. Also, in the method for determining HLA-A*24:02 of the present invention, it is preferable to carry out PCR with the genomic DNA as a template, using a primer designed to ensure that PCR products are obtained when the genomic DNA does not include the HLA-A*24:02. Examples of the primer designed to ensure that PCR products are obtained when the PCR products are not obtained depending on the HLA-A*24:02-specific primers for typing the bases of (c) (that is, primers that hybridize specifically with alleles in which the 437th base is C, the 441st base is T, the 443rd base is G, the 444th base is C, the 447th base is T, and the 449th base is C) include the primers-9 to -12 shown in Table 10.

**[Table 10]**

| Primer | Base sequence (5' → 3') | SEQ NO: |
|---|---|---|
| Primer-9 | GGTTGTAGTAGCCGCGCAGGGTCCC | 11 |
| Primer-10 | TAGCCGCGCAGGGTCCCCAGG | 12 |
| Primer-11 | TAGCCGCGCAGGGTCCCCAG | 13 |
| Primer-12 | CTCGCTCTGGTTGTAGTAGCCGCGCAGTG | 14 |

The determination method of the present invention can be carried out in a simpler manner by making the probe or primer used for the typing of the bases of (a) to (c) into a kit form. For example, a combination of the primers-1 to -4 shown in FIG. 3 can be used as a kit for determining an HLA-A*24 group. In the present invention, a kit for determining an HLA-A*24 group including the primer-5 that is an HLA-A*24:02-specific primer is preferred. A kit including the HLA-A*24:02-specific primer is suitably used for determining the HLA-A*24:02.

In addition, it is preferable that the kit for determining the HLA-A*24 group include a reagent, an instrument, or the like for extracting and purifying nucleic acids from a biological sample collected from a test subject.

### EXAMPLES

Hereinafter, the present invention will be described specifically with reference to Examples, but the present invention is not limited to the following Examples.

### [Example 1]

PCR using the primers-1 and -2, and PCR using the primers-3 and -4, shown in Table 8, were carried out, using a genomic DNA extracted from a biological sample collected from a test subject, in which the HLA-A type is a [A*24:02/A*24:02] homotype, a heterotype of [A*24:02/A*24:08], a heterotype of [A*24:20/A*32:01], and a heterotype of [A*32:01/A*24:04] as a template, and each of the PCR products was subjected to sequence analysis.

The determination results on the basis of the sequence analysis results and the typing results thus obtained are shown in Table 11. In the Table, in the column of "Determination", "+" denotes a determination that the genomic DNA includes HLA-A*24:02 and "-" denotes a determination that the genomic DNA does not include HLA-A*24:02. As a result, the [A*24:02/A*24:02] homotype, the heterotype of [A*24:02/A*24:08], and the heterotype of [A*32:01/A*24:04] were all accurately determined. On the other hand, the heterotype of [A*24:20/A*32:01] was false-positive, as expected.

**[Table 11]**

| | (a) | (b) | (c) | Type | Determination | Errata |
|---|---|---|---|---|---|---|
| A*24:02/A*24:02 | C | G | C | + | + | ○ |
| A*24:02/A*24:08 | A/C | C/G | C | + | + | ○ |
| A*24:20/A*32:01 | A/C | C/G | C | - | + | × |
| A*32:01/A*24:04 | C | C/G | C/G | - | - | ○ |

### [Example 2]

In the same manner as in Example 1 except that the primer-5 shown in Table 9 was used instead of the primer-4, PCR was carried out, using a genomic DNA extracted from a biological sample collected from a test subject, in which the HLA-A type is a [A*24:02/A*24:02] homotype, a heterotype of [A*24:02/A*24:08], a heterotype of [A*24:20/A*32:01], and a heterotype of [A*32:01/A*24:04], as a template. As a result, in all the cases where any genomic DNA was used as a template, PCR products were obtained by PCR, using the primers-3 and -5. Therefore, the genomic DNA of all the test subjects was typed such that the 437th base was C, the 441st base was T, the 443rd base was G, the 444th base was C, the 447th base was T, and the 449th base was C. As a result of the sequence analysis of each PCR product, the same determination results as those of Example 8, as seen from Table 12, were obtained. However, when using a genomic DNA derived from the heterotype of [A*32:01/A*24:04], only two types, C and G, of the bases of (b) were typed in Example 1, but in this Example, only one type, C, of the bases of (b) was typed.

**[Table 12]**

| | (a) | (b) | Type | Determination | Errata |
|---|---|---|---|---|---|
| A*24:02/A*24:02 | C | G | + | + | ○ |
| A*24:02/A*24:08 | A/C | C/G | + | + | ○ |
| A*24:20/A*32:01 | A/C | C/G | - | + | × |
| A*32:01/A*24:04 | C | C | - | - | ○ |

### Industrial Applicability

Since the determination method of the present invention makes it possible to determine the typing of the HLA-A*24 group with high precision in a simpler manner, it can be used in the fields of clinical genetic diagnostics, or the like, in particular, in the determination of an HLA type for cell or organ transplantation.

## Claims

1. A method for determining an HLA-A*24 group, comprising:
determining the HLA-A*24 group on the basis of the results of the typing of (a) a 211st base and (b) a 395th base, in which A (adenine) in the initiation codon for an HLA gene is defined as the 1st base in the genomic DNA of a test subject.

2. The method for determining an HLA-A*24 group according to claim 1, wherein the HLA-A*24 group is determined, further on the basis of the results of the typing of (c) at least one base selected from the group consisting of a 437th base, a 441st base, a 443rd base, a 444th base, a 447th base, and a 449th base.

3. The method for determining an HLA-A*24 group according to claim 2, wherein it is determined whether or not the HLA type of the test subject is HLA-A*24:02.

4. The method for determining an HLA-A*24 group according to claim 3, wherein when the 211st base is C (cytosine), the 395th base is G (guanine), the 437th base is C, the 441st base is T (thymine), the 443rd base is G, the 444th base is C, the 447th base is T, and the 449th base is C in at least one allele, the HLA type of the test subject is determined to be HLA-A*24:02.

5. The method for determining an HLA-A*24 group according to any one of claims 1 to 4, wherein
the typing of the bases of (a) and (b) is carried out by a sequence analysis method or a single-base detection method.

6. The method for determining an HLA-A*24 group according to any one of claims 2 to 5, wherein
the typing of the bases of (c) is carried out by a sequence analysis method or a single-base detection method.

7. The method for determining an HLA-A*24 group according to any one of claims 2 to 5, wherein
when an elongation product is obtained by an elongation reaction by a polymerase using a primer containing a base sequence represented by SEQ NO: 7, the bases of (c) are typed as follows; the 437th base is C, the 441st base is T, the 443rd base is G, the 444th base is C, the 447th base is T, and the 449th base is C.

8. A kit for determining HLA-A*24:02, comprising a primer for determining HLA-A*24:02 containing a base sequence represented by SEQ NO: 7.
